# EUROPÄISCHE PATENTSCHRIFT

(11) **EP 3 740 322 B1**
(45) Veröffentlichungstag und Bekanntmachung des Hinweises auf die Patenterteilung: **31.08.2022**
(21) Anmeldenummer: 19703946.4
(22) Anmeldetag: 16.01.2019
(51) Int. Cl.: B05B 11/04, B05B 11/00, A61F 9/00

(54) **VORRICHTUNG ZUR DOSIERUNG VON FLÜSSIGKEIT**
DEVICE FOR METERING A LIQUID
DISPOSITIF DE DOSAGE D'UN LIQUIDE

(30) Priorität: 16.01.2018 DE 102018100847; 12.04.2018 DE 102018108700; 23.07.2018 DE 102018117731
(43) Veröffentlichungstag der Anmeldung: 25.11.2020
(73) Patentinhaber: F+K Innovationen GmbH & Co.KG, 76534 Baden-Baden (DE)
(72) Erfinder: FUCHS, Karl-Heinz, 78315 Radolfzell (DE)
(74) Vertreter: Patentanwälte und Rechtsanwalt Weiß, Arat & Partner mbB
(86) Internationale Anmeldenummer: PCT/EP2019/051017
(87) Internationale Veröffentlichungsnummer: WO 2019/141713

(56) Entgegenhaltungen:
- WO-A1-2013/163364
- WO-A2-2008/036974
- DE-T2- 69 210 561
- FR-A1- 2 568 007
- FR-A1- 2 859 464
- GB-A- 2 462 136

## Beschreibung

### Technisches Gebiet

Die Erfindung betrifft eine Vorrichtung zur Dosierung von Flüssigkeit nach dem Oberbegriff des Anspruchs 1, bzw. des Anspruchs 2.

### Stand der Technik

Derartige Vorrichtungen zur Dosierung von Augentropfenflüssigkeit sind bereits in vielfältiger Form und Ausgestaltung bekannt und gebräuchlich. So wird beispielsweise in der WO 2017/162805 A1 eine solche Dosiervorrichtung offenbart, wobei dort in die Aussenwandung einer Flasche ein Luftventil eingelassen ist und im Inneren der Flasche ein Flüssigkeitsbeutel angeordnet ist.

Weiterhin offenbart die WO 2013/163364 A1 Fluidabgabevorrichtungen zum kostengünstigen Zuführen einer Flüssigkeit, z. B. eines Spülmediums, zu einer medizinischen Vorrichtung, z. B. einer Gefäßzugangsvorrichtung. Die Fluidabgabevorrichtung umfasst einen zusammenlegbaren Körper, der ein Fluidreservoir und einen Verbinder zum Befestigen der Fluidabgabevorrichtung an der medizinischen Vorrichtung definiert. Der zusammenklappbare Körper kann einen Balg oder eine gefaltete Struktur aufweisen. Zusätzlich kann eine Struktur einschließlich Hebeln oder Kolben vorgesehen werden, um den Körper zusammenzufallen.

Die FR 2 568 007 A1 offenbart eine Dosierflasche mit einem Behälter 1, der in ein Parallelepiped eingeführt ist. Zwei seiner gegenüberliegenden Seitenflächen können unter Verwendung eines Mittelteils zusammengedrückt werden, das mit den vier anderen benachbarten Flächen durch einen elastischen Balg verbunden ist, der diesen Mittelteil vollständig umgibt. Zwei starre Schalen verschließen die komprimierbaren Flächen mit einem Spiel, so dass sie durch Drücken zusammengebracht werden können und das Innenvolumen des Behälters verringert werden kann.

Die GB 2 462 436 A offenbart einen Flüssigkeitsapplikator, welcher ein Reservoir zum Aufnehmen einer aufzutragenden Flüssigkeit und einen mit dem Reservoir verbundenen Auslass umfasst. Zum Ablassen der Flüssigkeit ist eine Applikatorspitze vorgesehen. In der Mündung des Reservoirs befindet sich ein Stopfen, wobei der Stopfen und die Mündung so konfiguriert sind, dass der Stopfen von einer ersten Position, in der er das Reservoir abdichtet, zu einer zweiten Position bewegbar ist, in der Flüssigkeit aus dem Reservoir herausfließen kann.

Die DE 692 10 561 T2 betrifft einen Dosierspender, insbesondere für flüssige und viskose Produkte.

Ausserdem offenbart die FR 2 859 464 A1 eine Vorrichtung zum Verteilen eines flüssigen oder pastösen Produkts, z.B. eines Medikaments, mit einem Kraftübertragungsteil mit in Bewegungsrichtung des zylindrischen Körpers voneinander beabstandeten Scharnierfolien und relativ zur Trägereinheit geneigten Schwenkarmen zum Falten

In der WO 2008/036974 A2 wiederum ist ein Flüssigkeitsspender offenbart, der eine Speicherkammer zum Speichern einer Flüssigkeit, eine Sammelanordnung zum Sammeln eines Flüssigkeitsabschnitts, eine Pumpenanordnung und ein Rohr aufweist, das sich von innerhalb der Speicherkammer zu innerhalb der Sammelkammer erstreckt, durch die der Flüssigkeitsabschnitt gepumpt wird in die Sammelbaugruppe. Die Pumpenanordnung umfasst einen oder mehrere Pumpenaktuatoren, und der Flüssigkeitsspender kann mehrere Lagerkammern aufweisen, von denen jede ein entsprechendes Rohr aufweist.

### Aufgabe der Erfindung

Die Aufgabe der vorliegenden Erfindung ist es, die Nachteile aus dem Stand der Technik zu überwinden. Insbesondere soll eine Vorrichtung bereitgestellt werden, die eine angemessene Dosierung ermöglicht und dabei verhindert, dass beim Betätigen keine Strahlbildung der ausgebrachten Flüssigkeit entstehen kann.

### Lösung der Aufgabe

Zur Lösung der Aufgabe führen die Merkmale nach dem Anspruch 1, bzw. nach dem Anspruch 2.

Vorteilhafte Ausgestaltungen sind in den Unteransprüchen beschrieben.

Eine erfindungsgemässe Vorrichtung zur Dosierung von Flüssigkeit dient dem Ausbringen von Flüssigkeit aus einer Flüssigkeitsaufnahme. Flüssigekitsaufnahme ist hier eine Kunststoff-Flasche, die zusammengedrückt werden kann. Die Flüssigkeitsaufnahme umfasst ein Auslassventil. Das Auslassventil ist derart gestaltet, dass die Flüssigkeit ausgebracht werden kann, ohne dass dabei Luft durch das Auslassventil in die Flüssigkeitsaufnahme gelangen kann.

Hierzu ist ein Dosiersystem vorhanden, wobei das Dosiersystem aus einem ersten Betätigungsflügel und einem zweiten Betätigungsflügel oder eine geschlitzte Hülse um die Flüssigkeitsaufnahme angeordnet sind und zueinander die Flüssigkeitsaufnahme komprimierend bewegbar sind, wobei ein mechanischer Dosiereinschlag vorhanden ist. Der mechanische Dosieranschlag ist hierbei derart vorgesehen, dass die beiden Betätigungsflügel nur in definiertem Umfang zueinander bewegbar sind, bzw. bei der geschlitzten Hülse beide Hälften zueinander definiert bewegbar sind.

Der erste Betätigungsflügel weist eine erste Fingerauflage am Fingerbetätigungsflansch auf und der zweite Betätigungsflügel weist eine Fingerauflage am Fingerbetätigungsflansch auf. Diese Fingerauflagen dienen der besseren Nutzung des Dosiersystems sowie zur Betätigungskraftreduktion, indem über die Betätigungsflügel der Betätigungsweg verlängert wird.

Weiter umfasst der erste Betätigungsflügel einen ersten Verbindungssteg und der zweite Betätigungsflügel umfasst einen zweiten Verbindungssteg. Dabei können in bevorzugten Ausführungsbeispielen zwei erste Verbindungsstege und zwei zweite Verbindungsstege angeordnet sein, die dann die Flüssigkeitsaufnahme umgreifen. Die Betätigungsflügel der geschlitzten Hülse können auch ohne Verbindungsstege ausgebildet sein.

Der erste Verbindungssteg weist einen Führungsstift auf und der zweite Verbindungssteg weist ein Langloch auf. Es handelt sich hierbei um einen mechanischen Dosieranschlag, da der Führungsstift in dem Langloch definiert geführt wird und bei Erreichen des Endes des Langloches handelt es sich dabei um einen mechanischen Anschlag. Desweiteren kann auch ein mechanischer Anschlag an den Flügeln bzw. Halbschalen angeordnet sein.

Das System wird immer über die komprimierte Luft betätigt.

Wenn das Luftventil bzw. der Ventilverschluss gut funktioniert, sollte immer eine Luftmenge zwischen Beutel und Flaschenwandung eingeschlossen sein. D.h. der Betätigungshub ist ungefähr immer gleich. Das bedeutet, dass Luftmengenänderung betreffend der Beutelentleerung unberücksichtigt bleibt.

Um eine gleichbleibende Tropfendosierung ohne Strahlbildung zu erzeugen, würde ein Betätigungsweganschlag ausreichen, d.h. Hubbegrenzung für eine Tropfengröße.

Mit einem Betätigungsflügel, also einer der Betätigungshalbschale, welche an der Flasche in Form der Flüssigkeitsaufnahme verankert ist und eine Betätigungswegbegrenzung hat, kann eine gleichbleibende Tropfendosierung beim Betätigen der Betätigungshalbschalen erzeugt werden sowie die Betätigungskraft reduziert und die Strahlbildung verhindert werden.

Weiter umfasst der erste Betätigungsflügel einen ersten Verbindungssteg und der zweite Betätigungsflügel umfasst einen zweiten Verbindungssteg. Dabei können in bevorzugten Ausführungsbeispielen zwei erste Verbindungsstege und zwei zweite Verbindungsstege angeordnet sein, die dann die Flüssigkeitsaufnahme umgreifen.

Der erste Verbindungssteg weist einen Führungsstift auf und der zweite Verbindungssteg weist ein Langloch auf. Es handelt sich hierbei um einnen mechanischen Dosieranschlag, da der Führungsstift in dem Langloch definiert geführt wird und bei Erreichen des Endes des Langloches handelt es sich dabei um einen mechanischen Anschlag.

Das System wird immer über die komprimierte Luft betätigt.

Um eine gleichbleibende Tropfendosierung ohne Strahlbildung zu erzeugen, würde ein Betätigungsweganschlag ausreichen d.h. Hubbegrenzung für eine Tropfengröße.

Aus fertigungstechnischen Gründen kann der Anschlag nicht in das Flascheninnere integriert werden.

Mit einer der Betätigungsflügel also einer der Betätigungshalbschalen, welche an der Flasche in Form der Flüssigkeitsaufnahme verankert ist und eine Betätigungswegbegrenzung hat, kann eine gleichbleibende Tropfendosierung beim Betätigen der Betätigungshalbschale erzeugt werden sowie die Betätigungskraft reduziert und die Strahlbildung verhindert werden.

Damit das System vor ungewolltem Betätigen geschützt ist, hat die Schutzklappe zwei Taschen, in welche die Fingerbetätigungsflansche bei aufgesetzter Schutzkappe greifen und somit bei aufgesteckter Schutzkappe ein Betätigen verhindern. Dieser Bereich kann auch mit einem kindersicheren Verschluss kombiniert werden.

Die Betätigungsbügel oder die geschlitzte Hülse kann zur Flüssigkeitsaufnahme konturvorgespannt oder nur in verschnappter bzw. in der Kombination beider Varianten angeordnet sein, wobei sich der Fingerbetätigungsbereich am Flansch verwindungsfrei mit Betätigungsspiel zur Flüssigkeitsaufnahme angeordnet ist, um den Betätigungsweg zu ermöglichen.

Als zusätzliche Demontagesicherheit besitzt die Flüssigkeitsaufnahme kleine Vertiefungen, in welche die Betätigungsflügel bei der Montage der Betätigungsflügel mit der Flüssigkeitsaufnahme eingreifen.

Um dem Betätigungsflügel den Betätigungsweg zu ermöglichen, sind beide Flügel oder Halbschalen gegenüber des Betätigungsbereiches mit einem Filmscharnier bzw. mit einer Scharniergeometrie verbunden.

Um das separate Luftventil in der Flüssigkeitsaufnahme zu ersetzen, ist ein konischer rundere Zapfen, welcher an der Betätigungsflügelinnenwandung angeordnet ist und beim Betätigen der Flügel sich in das Belüftungsloch der Flüssigkeitsaufnahmenwandung bewegt und über die konische Formgebung das System gegen das Austreten der Luft verschließt, so dass zwischen Innenbeutel und Flüssigkeitsaufnahmewand die eingeschlossene Luft komprimiert werden kann und dadurch, durch die Betätigung der Betätigungsflügel das Medium über das Auslassventil austreten kann, eingesetzt werden.

Mit diesem Systemaufbau wird ein teilereduziertes Tropfendosiersystem bei geringer Betätigungskraft als Airless-System ohne atmosphärische Zugangsöffnungen in einfacher Bauweise generiert.

In einem weiteren Ausführungsbeispiel der erfindungsgemässen Vorrichtung zur Dosierung von Flüssigkeit aus einer Flüssigkeitsaufnahme und der Flüssigkeitsaufnahme ein Auslassventil zugeordnet ist. Weiter ist ein Dosiersystem vorhanden, wobei das Dosiersystem wiederum aus dem ersten Betätigungsflügel und dem zweiten Betätigungsflügel besteht, welche um die Flüssigkeitsaufnahme angeordnet sind und zueinander die Flüssigkeitsaufnahme komprimierend bewegbar sind, wobei der erste Betätigungsflügel einen ersten Druckkolben aufweist und der zweite Betätigungsflügel einen zweiten Druckkolben aufweist, wobei eine Ausbringkammer durch die zueinander bewegbar gelagerten Druckkolben ausdrückbar angeordnet ist und die beiden Druckkolben durch ein Federelement und/oder Teilfedern rückstellbar angeordnet sind. Hier ist eine besonders gute und einfache Dosierung möglich.

### Figurenbeschreibung

Weitere Vorteile, Merkmale und Einzelheiten der Erfindung ergeben sich aus der nachfolgenden Beschreibung bevorzugter Ausführungsbeispiele sowie anhand der Zeichnungen; diese zeigen in:
Figur 1 eine Vorrichtung mit Schutzkappe, wobei die Vorrichtung nicht unter die Ansprüche fällt;
Figur 2 ein Vorrichtung ohne Schutzkappe, wobei die Vorrichtung nicht unter die Ansprüche fällt;
Figur 3 eine Vorrichtung mit dem Fingerbetätigungsflansch, wobei die Vorrichtung nicht unter die Ansprüche fällt;
Figur 4 eine Vorrichtung mit geschlitzter Hülse, wobei die Vorrichtung nicht unter die Ansprüche fällt;
Figur 5 eine erfindungsgemäße Vorrichtung mit den Verbindungsstegen;
Figur 6 eine erfindungsgemäße Vorrichtung des Dosiersystems;
Figur 7 eine Vorrichtung mit den Verbindungsstegen und Betätigungsanschlag, wobei die Vorrichtung nicht unter die Ansprüche fällt;
Figur 8 eine Vorrichtung mit geschlitzter Hülse ohne Betätigungsflansch, wobei die Vorrichtung nicht unter die Ansprüche fällt;
Figur 9 eine Vorrichtung mit Verbindungsstegen, wobei die Vorrichtung nicht unter die Ansprüche fällt;
Figur 10 eine Vorrichtung ohne Verbindungsstegen, wobei die Vorrichtung nicht unter die Ansprüche fällt;
Figuren 11 bis 13 eine erfindungsgemäße Vorrichtung gemäß einem weiteren Ausführungsbeispiel.

### Ausführungsbeispiel

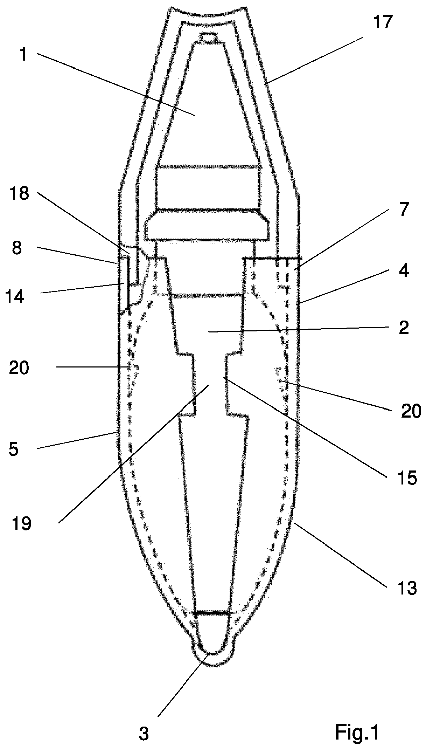

In Fig.1 ist eine Vorrichtung in Ansicht und teils im Schnitt dargestellt. Die Flüssigkeitsaufnahme 2 in der konturvorgespannten geschlitzten Hülse 13 mit den beiden Betätigungsflügel 4 und 5 , welche über ein Filmscharnier 3 beweglich bis zum Hubanschlag 15 verbunden sind. Die Aussparungen 20 in der Flüssigkeitsaufnahme 2 nehmen die konturvorgespannte geschlitzte Hülse 13 zur Demontagesicherung auf.

Zum Betätigungsschutz des Systems sitzt auf dem Auslassventil 1 eine Schutzkappe 17 mit zwei seitlichen Taschen 18 zur Aufnahme der Fingerbetätigungsflansche 14 mit der Fingerauflage 7 und 8, um das ungewollte Betätigen des Systems zu verhindern.

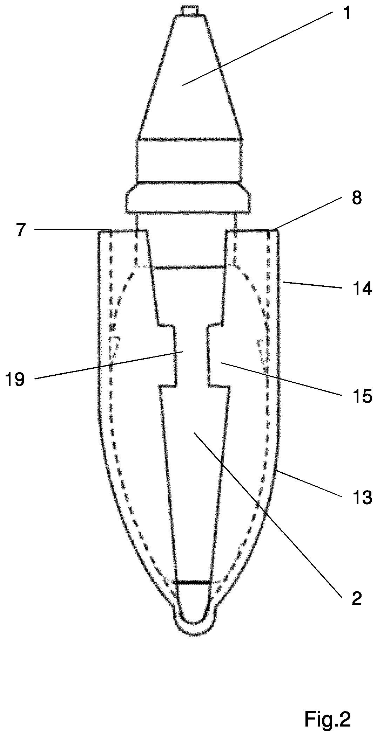

Fig.2 zeigt eine Vorrichtung in Ansicht ohne Schutzkappe 17. Die geschlitzte konturvorgespannte Hülse 13 ummantelt die Flüssigkeitsaufnahme 2. Am oberen Ende der geschlitzten Hülse 13 sitzen die beiden Fingerbetätigungsflansche 14 mit der Fingerauflage 7 und 8 als Betätigungselemente für das System.

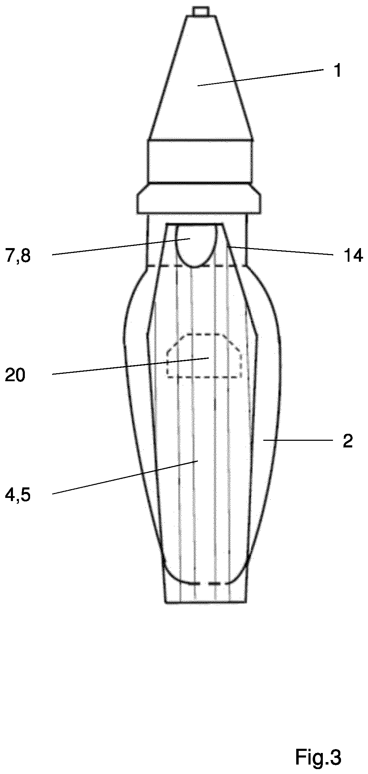

Fig.3 zeigt eine Vorrichtung als Ansicht mit dem Fingerbetätigungsflansch 14, welcher mit den Betätigungsflügeln 4 und 5 über die Aussparung 20 die Flüssigkeitsaufnahme 2 konturvorgespannt ummanteln sowie die Fingerauflagen 7 und 8.

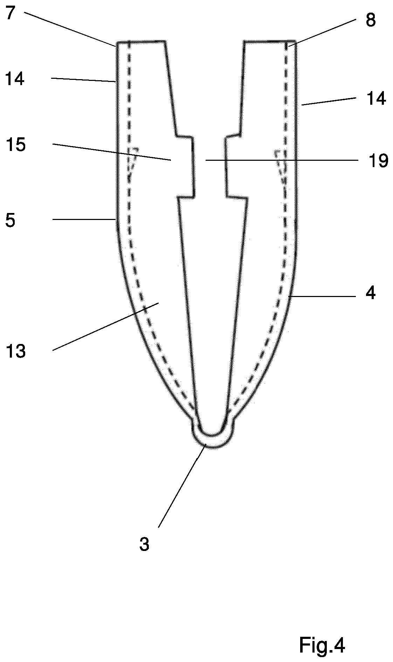

Fig.4 zeigt die geschlitzte Hülse 13 mit den beiden Betätigungsbügeln 4 und 5, dem Filmscharnier 3 sowie die Betätigungsflansche 14 mit der Fingerauflage 7 und 8.

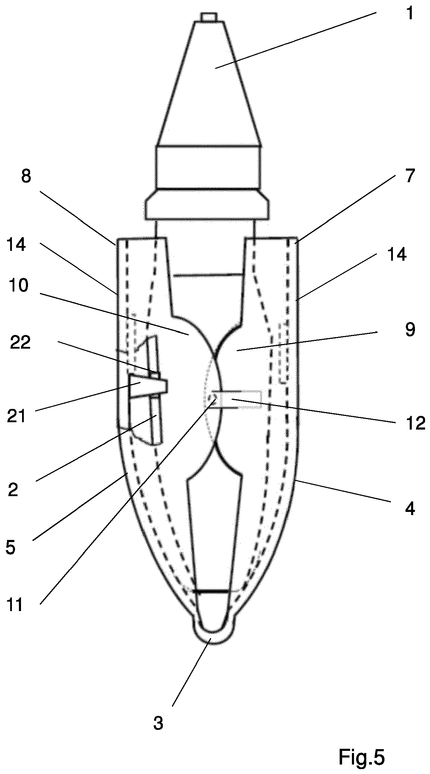

Fig.5 zeigt eine erfindungsgemäße Vorrichtung in Ansicht ohne Schutzkappe 17 als verschnappbare Ausführung. Die Betätigungsbügel 4 und 5, welche über das Filmscharnier 3 beweglich verbunden sind, werden über Verbindungsstege 9 und 10 durch den Führungsstift 11, welcher in einem Langloch 12 des zweiten Verbindungsstegs 10 angeordnet ist, miteinander fixiert und beweglich gehalten. Auf der gegenüber liegenden Seite dieser Ansicht ist symmetrisch die gleiche Anordnung integriert. Im Teilschnitt ist der konisch runde Zapfen 21 , welcher sich an der Innenwand 6 des Betätigungsbügels 4 oder 5 befindet, dargestellt, welcher beim Betätigen über die Fingerbetätigungsflansche 14 mit der Fingerauflage 7 und 8 das Belüftungsloch 22 in der Flüssigkeitsaufnahmenwand 2 verschließt und in der Ausgangsposition das Loch zum Belüften wieder freigibt.

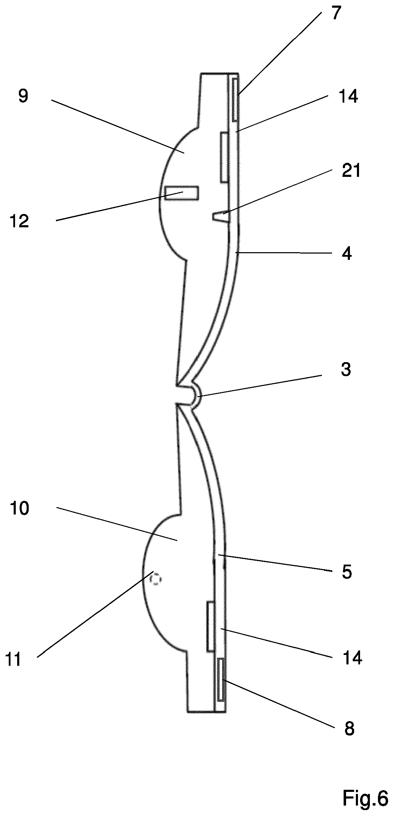

In Fig.6 wird das verschnappte System in aufgeklapptem Zustand gezeigt. Die beiden Betätigungsbügel 4 und 5, welche über das Filmscharnier 3 beweglich verbunden sind, gehen über in den Fingerbetätigungsflansch 14, welcher die Fingerauflage 7 und 8 beinhaltet. Die Verbindungsstege 9 und 10 sowie den Führungsstift 11 und das Langloch 12 sind im Fingerbetätigungsflansch 14 integriert.

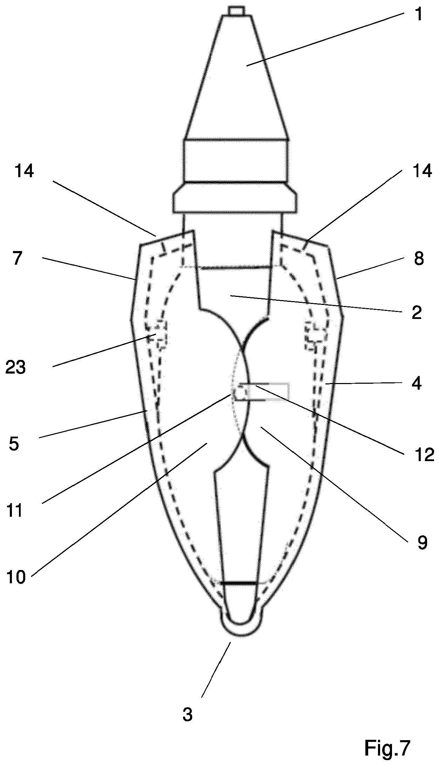

Fig.7 zeigt eine Vorrichtung in Ansicht ohne Schutzkappe 17 als verschnappbare Ausführung. Die Betätigungsbügel 4 und 5, welche über das Filmscharnier 3 beweglich verbunden sind, werden über Verbindungsstege 9 und 10 durch den Führungsstift 11, welche in ein Langloch 12 des Verbindungsstegs 10 miteinander fixiert und in Position gehalten. Die Betätigungsbügel 4 und 5 greifen über runde Elementzapfen 23 in die Flüssigkeitsaufnahme 2 und fixieren das System.

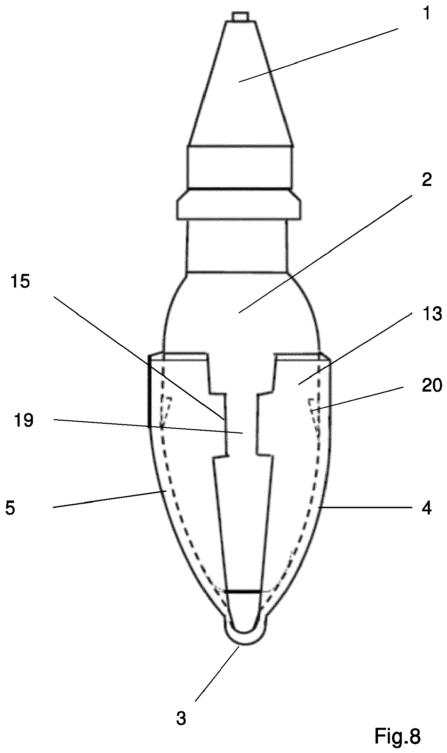

Fig.8 zeigt eine Vorrichtung ohne Schutzkappe 17. Bei dieser Ausführung ist die geschlitzte vorgespannte Hülse 13 ohne Führungsbetätigungsflansch 14 dargestellt, so dass die Betätigung direkt über die Hülsenwandung erfolgt.

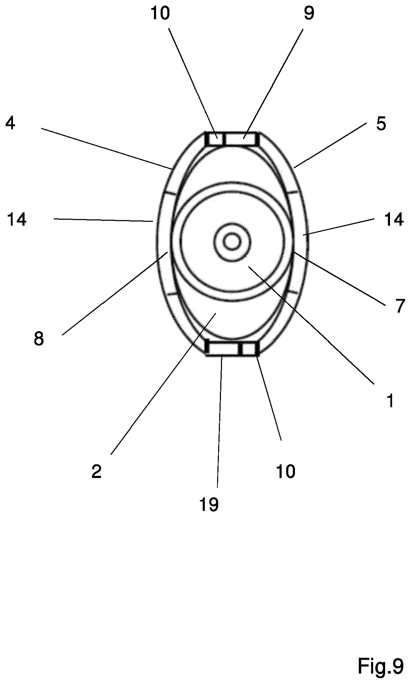

Fig.9 zeigt die Vorrichtung als verschnappbare Ausführung in der Draufsicht mit den beiden Verbindungsstegen 9 und 10, den beiden Fingerbetätigungsflansche 14 mit den Fingerauflagen 7 und 8 sowie mit dem Auslassventil 1 mit der Flüssigkeitsaufnahme 2.

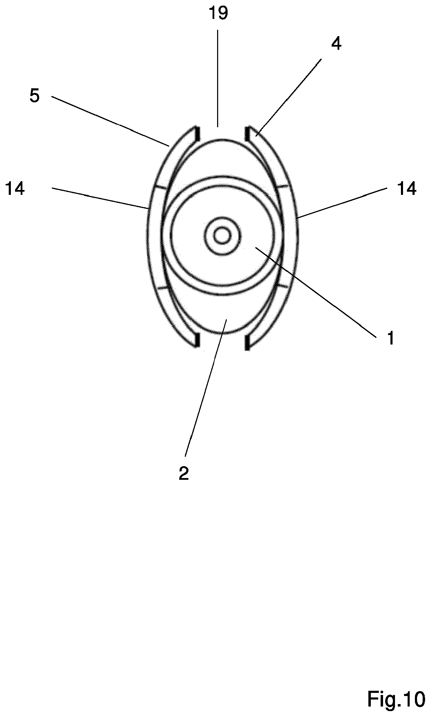

Fig.10 zeigt die Vorrichtung als konturvorgespannte Ausführung in der Draufsicht mit den beiden Fingerbetätigungsflansche 14, den Betätigungsbügeln 4 und 5 sowie das Auslassventil 1 mit der Flüssigkeitsaufnahme 2. Der Betätigungsweg 19 liegt zwischen den Betätigungsbügeln 4 und 5.

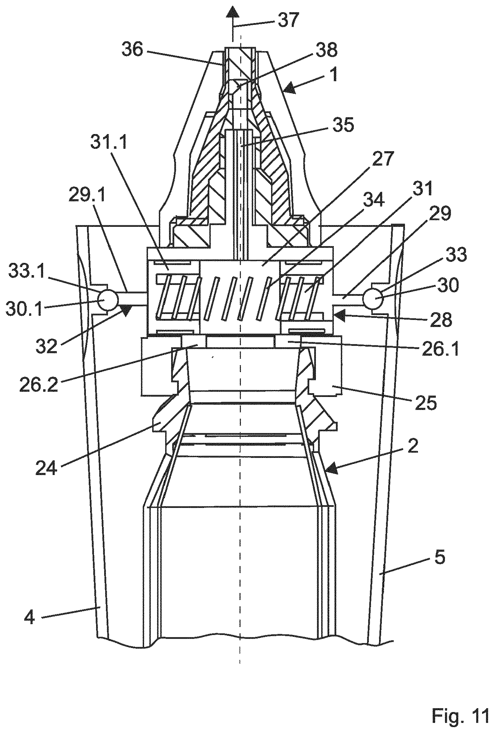

In Fig. 11 ist eine geschnittene Seitenansicht eines Teils eines weiteren Ausführungsbeispiels gezeigt. Dort sind wieder die beiden Betätigungsflügel 4, 5 zu erkennen. Zwischen den beiden Betätigungsflügeln 4, 5 ist die Flüssigkeitsaufnahme 2 angeordnet. Die Flüssigkeitsaufnahme 2 weist einen Halsclip 24 auf. Auf dem Halsclip 24 ist ein Ventilclip 25 gegeben. Der Ventilclip 25 ist auf den Halsclip 24 aufclipsbar und verbindet somit das Ventil 1 mit der Flüssigkeitsaufnahme 2. Dazu weist der Ventilclip 25 zwei Durchbrüche 26.1, 26.2 auf, wobei durch die beiden Durchbrüche 26.1, 26.2 das Medium aus der Flüssigkeitsaufnahme 2 in eine Ausbringkammer 27 drückbar ist.

Das Ventil 1 weist eine Dichtlippe 36 auf welche das Medium aus einem Ausbringkanal 35 nach aussen abgibt, wie es der Pfeil 37 andeutet. Dabei weist der Ausbringkanal 35 im Bereich der Dichtlippe 36 eine 90°-Kehre 38 hin zur Dichtlippe 36 auf.

Weiter umfasst der erste Betätigungsflügel 4 einen ersten Druckkolben 28 auf. Der erste Druckkolben 28 wiederum besteht aus einem Stößel 29, einem Gelenkkopf 30 und einem Komprimierelement 31. In gleicher Weise ist ein zweiter Druckkolben 32 gezeigt, welcher wiederum aus einem Stößel 29.1, einem Gelenkkopf 30.1 und einem Komprimierelement 31.1 besteht.

Der Gelenkkopf 30 wiederum ist in einer Gelenkaufname 33 aufgenommen, wobei die Gelekaufnahme 33 als Teil des zweiten Betätigungsflügels 5 ausgebildet. In Arbeitslage ist die Gelenkaufnahme 33 hin zur Flüssigkeitsaufnahme 2 hin ausgebildet. Arbeitslage bedeutet hierbei, wenn die Flüssigkeitsaufnahme 2 zwischen den beiden Betätigungsflügeln 4, 5 angeordnet ist und das Medium herausgedrückt werden kann.

Ausserdem ist der Gelenkkopf 30.1 wiederum ist in einer Gelenkaufnahme 33.1 aufgenommen, wobei die Gelenkaufnahme 33.1 als Teil des ersten Betätigungsflügels 4 ausgebildet. In Arbeitslage ist die Gelekaufnahme 33.1 hin zur Flüssigkeitsaufnahme 2 hin ausgebildet.

Weiter ist ein Federelement 34 gezeigt, welches zwischen dem ersten Druckkolben 28 und dem zweiten Druckkolben 32 angeordnet. Nachdem die beiden Druckkolben 28, 32 durch die beiden Betätigungsflügel 4, 5 zueinander bewegt wurden und die beiden Komprimierelemente 31, 31.1 die Ausbringkammer 27 zueinander durchschritten haben, um das dort befindliche Medium in einen Ausbringkanal 35 zu drücken, stellt das Federlement 34 die beiden Komprimierlemente 31, 31.1 in die Ausgangslage zurück.

Anschliessend entsteht in der Ausbringkammer 27 ein Unterdruck und durch die beiden Durchbrüchen 26.1, 26.2 kann neues Medium aus der Flüssigkeitsaufnahme 2 in die Ausbringkammer 27 fließen. In diesem Ausführungsbeispiel ist das einzige Federelement 34 zwischen dem ersten Druckkolben 28 und dem zweiten Druckkolben 32 angeordnet.

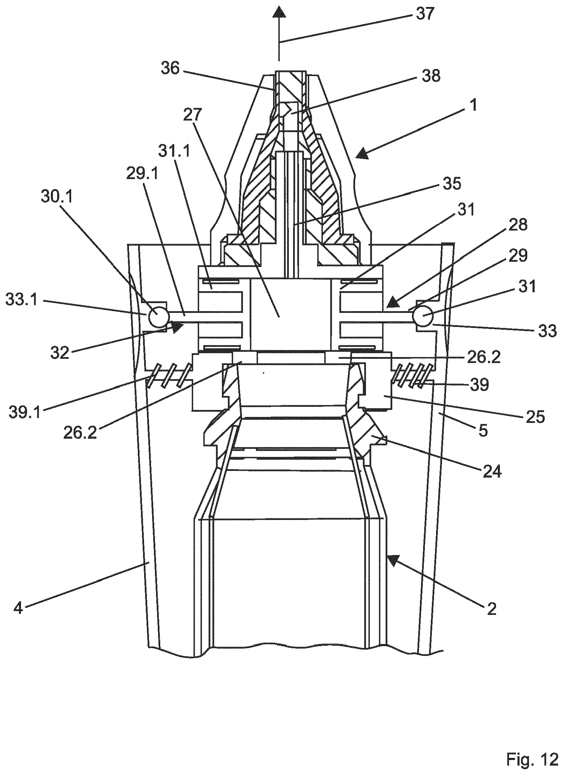

In Figur 12 ist ein weiteres Ausführungsbeispiel gezeigt, wobei die Ausführungen zu den Merkmalen in den vorheriigen Figuren auch auf diese Figur lesbar sein sollen. Insbesondere gilt dies bei Merkmalen mit identischen Bezugsziffern. Auf eine ausdrückliche Wiederholung wird hier verzichtet.

Anders als bei der Figur 11 sind hier zwei Teilfedern 39, 39.1 gezeigt. Dabei ist die erste Teilfeder 39 zwischen dem zweiten Betätigungsflügel 5 und dem Ventilclip 25 angeordnet. Weiter ist die zweite Teilfeder 39.1 zwischen dem ersten Betätigungsflügel 4 und dem Ventilclip 25 angeordnet.

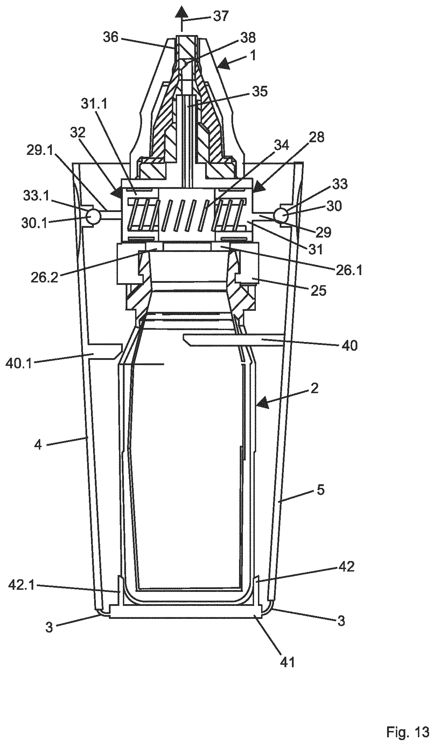

In Figur 13 ist ein weiteres Ausführungsbeispiel gezeigt, wobei die Ausführungen zu den Merkmalen in den vorheriigen Figuren auch auf diese Figur lesbar sein sollen. Insbesondere gilt dies bei Merkmalen mit identischen Bezugsziffern. Auf eine ausdrückliche Wiederholung wird hier verzichtet.

Ansonsten ist ein erster Haltestift 40.1 gezeigt. Der erste Haltestift 40.1 ragt in Arbeitslage von dem zweiten Betätigungsflügel 5 weg in Richtung der Flüssigkeitsaufnahme 2. Weiter ist ein zweiter Haltestift 40.2. gezeigt, welcher wiederum in Arbeitslage von dem ersten Betätigungsflügel 4 weg hin zu der Flüssigkeitsaufnahme 2 angeordnet ist. Dabei reichen beide Haltestifte 40, 401 auch teilweise über die Flüssigkeitsaufnahme 2 hinweg. Weiter sind zwei Filmscharniere 3 gezeigt, welche einen Boden 41 jeweils mit den beiden Betätigungsflügeln 4, 5 verbinden. Weiter bildet der Boden 41 einen ersten Bodenstift 42 und einen zweiten Bodenstift 42.1 aus, um in Arbeitslage die Flüssigkeitsaufnahme 2 besser zu haltern.

Obwohl nur ein bevorzugtes Ausführungsbeispiel der Erfindung beschrieben und dargestellt wurde, ist es offensichtlich, dass der Fachmann zahlreiche Modifikationen hinzufügen kann, solange diese vom Umfang der Ansprüche abgedeckt sind.

### Bezugszeichenliste

| | |
|---|---|
| 1 | Auslassventil |
| 2 | Flüssigkeitsaufnahme |
| 3 | Filmscharnier |
| 4 | Erster Betätigungsflügel |
| 5 | Zweiter Betätigungsflügel |
| 6 | Innenwandung |
| 7 | Erste Fingerauflage |
| 8 | Zweite Fingerauflage |
| 9 | Erster Verbindungssteg |
| 10 | Zweiter Verbindungssteg |
| 11 | Führungsstift |
| 12 | Langloch |
| 13 | geschlitzte Hülse |
| 14 | Fingerbetätigungsflansch |
| 15 | Hubanschlag |
| 16 | Halbschale |
| 17 | Schutzkappe |
| 18 | Taschen |
| 19 | Betätigungsweg |
| 20 | Aussparung |
| 21 | konisch runder Zapfen |
| 22 | Belüftungsloch |
| 23 | Elementzapfen |
| 24 | Halsclip |
| 25 | Ventilclip |
| 26 | Durchbruch |
| 27 | Ausbringkammer |
| 28 | Erster Druckkolben |
| 29 | Stößel |
| 30 | Gelenkkopf |
| 31 | Komprimierelement |
| 32 | Zweiter Druckkolben |
| 33 | Gelenkaufnahme |
| 34 | Federelement |
| 35 | Ausbringkanal |
| 36 | Dichtlippe |
| 37 | Pfeil |
| 38 | 90°-Kehre |
| 39 | Teilfeder |
| 40 | Haltestift |
| 41 | Boden |

## Patentansprüche

1. Vorrichtung zur Dosierung von Flüssigkeit aus einer Flüssigkeitsaufnahme (2), wobei die Flüssigkeitsaufnahme (2) ein Auslassventil (1) umfasst,
wobei
ein Dosiersystem vorhanden ist, wobei das Dosiersystem aus einem ersten Betätigungsflügel (4) und einem zweiten Betätigungsflügel (5) besteht, welche um die Flüssigkeitsaufnahme (2) angeordnet werden können und zueinander die Flüssigkeitsaufnahme (2) komprimierend bewegbar sind, **dadurch gekennzeichnet, dass** der erste Betätigungsflügel (4) einen ersten Druckkolben (28) aufweist und der zweite Betätigungsflügel (5) einen zweiten Druckkolben (32) aufweist, wobei eine Ausbringkammer (27) durch die zueinander bewegbar gelagerten Druckkolben (28, 32) ausdrückbar angeordnet ist und die beiden Druckkolben (28, 32) durch ein Federelement (34) und/oder Teilfedern (39, 39.1) rückstellbar angeordnet sind.

2. Vorrichtung zur Dosierung von Flüssigkeit aus einer Flüssigkeitsaufnahme (2), wobei die Flüssigkeitsaufnahme (2) ein Auslassventil (1) umfasst,
wobei ein Dosiersystem vorhanden ist, wobei das Dosiersystem aus einem ersten Betätigungsflügel (4) und einem zweiten Betätigungsflügel (5) besteht, welche um die Flüssigkeitsaufnahme (2) angeordnet werden können und zueinander die Flüssigkeitsaufnahme (2) komprimierend bewegbar sind, wobei ein mechanischer Dosieranschlag vorhanden ist, wobei die Flüssigkeitsaufnahme (2) durch eine geschlitzte konturvorgespannte Hülse (13) der Vorrichtung ummantelt ist, wobei
der erste Betätigungsflügel (4) und der zweite Betätigungsflügel (5) eine Innenwand (6) aufweisen, wobei der erste Betätigungsflügel (4) eine erste Fingerauflage (7) und der zweite Betätigungsflügel (5) eine zweite Fingerauflage (8) aufweisen, wobei am oberen Ende der geschlitzten Hülse (13) zwei Fingerbetätigungsflansche (14) angeordnet sind, **dadurch gekennzeichnet, dass** ein konisch runder Zapfen (21) vorhanden ist, welcher sich an der Innenwand (6) der Betätigungsflügel (4, 5) befindet, wobei bei Betätigen über die Fingerbetätigungsflansche (14) mit der Fingerauflage (7, 8) ein Belüftungsloch (22) in einer Wandung der Flüssigkeitsaufnahme (2) verschließt und in der Ausgangsposition das Loch zum Belüften wieder freigibt.

3. Vorrichtung nach Anspruch 1 oder 2, **dadurch gekennzeichnet, dass** der erste Betätigungsflügel (4) und der zweite Betätigungsflügel (5) über ein Filmscharnier (3) verbunden sind und zueinander verschwenkbar sind.

4. Vorrichtung nach einem der vorigen Ansprüche 1 bis 3, **dadurch gekennzeichnet, dass** der erste Betätigungsflügel (4) einen ersten Verbindungssteg (9) und oder einen zweiten Verbindungssteg (10) umfasst, wobei der zweite Betätigungsflügel (5) den ersten Verbindungssteg (9) und/oder den zweiten Verbindungssteg (10) umfasst.

5. Vorrichtung nach Anspruch 4, wenn abhängig vom Anspruch 2, **dadurch gekennzeichnet, dass** der erste Verbindungssteg (9) einen Führungsstift (11) aufweist und der zweite Verbindungssteg (10) ein Langloch (12) aufweist.

6. Vorrichtung nach einem der vorigen Ansprüche 1 bis 5, **gekennzeichnet durch** eine Schutzkappe (17) mit Taschen (18).

7. Vorrichtung nach einem der vorigen Ansprüche 1 bis 6, wenn abhängig vom Anspruch 2, **dadurch gekennzeichnet, dass** die Betätigungsflügel (4, 5) über eine Aussparung (20) die Flüssigkeitsaufnahme (2) konturvorgespannt ummanteln.

8. Vorrichtung nach den Ansprüchen 2, 3 und 5, **dadurch gekennzeichnet, dass** die Betätigungsbügel (4, 5), welche über das Filmscharnier (3) beweglich verbunden sind, über die Verbindungsstege (9, 10) durch den Führungsstift (11), welcher in dem Langloch (12) des zweiten Verbindungsstegs (10) angeordnet ist, miteinander fixiert und beweglich gehalten sind.

## Claims

1. Device for dosing liquid from a liquid intake (2), wherein the liquid intake (2) comprises an outlet valve (1), wherein a dosing system is provided, wherein the dosing system comprises a first actuating blade (4) and a second actuating blade (5), which can be arranged around the liquid intake (2) and are movable can be moved towards each other compressing the liquid receptacle (2),
**characterized in that**
the first actuating blade (4) has a first pressure piston (28) and the second actuating blade (5) has a second pressure piston (32), wherein an output chamber (27) is arranged so as to be able to be pressed out by the pressure pistons (28, 32) which are movably mounted relative to one another, and the two pressure pistons (28, 32) are resettable arranged by a spring element (34) and/or partial springs (39, 39.1).

2. Device for dosing liquid from a liquid intake (2), wherein the liquid intake (2) comprises an outlet valve (1), wherein a dosing system is provided, wherein the dosing system comprises a first actuating blade (4) and a second actuating blade (5) which can be arranged around the liquid intake (2) and are movable can be moved towards each other compressing the liquid receptacle (2), wherein a mechanical dosing stop is provided, wherein the liquid intake (2) is encased by a contour pre-stressed slotted sleeve (13) of the device, wherein the first actuating blade (4) and the second actuating blade (5) have an inner wall (6), the first actuating blade (4) having a first finger support (7) and the second actuating blade (5) having a second finger support (8). wherein two finger actuating flanges (14) are arranged at the upper end of the slotted sleeve (13), **characterised in that** a conically round pin (21) is provided, which is located on the inner wall (6) of the actuating blades (4, 5), wherein upon actuation via the finger actuating flanges (14) with the finger supports (7, 8) a vent hole (22) in a wall of the liquid intake (2) is closed (7, 8) and in the starting position releases the hole again for ventilation.

3. Device according to claim 1 or 2, **characterized in that** the first actuating blade (4) and the second actuating blade (5) are connected via a film hinge (3) and are pivotable relative to one another.

4. Device according to one of the previous claims 1 to 3, **characterized in that** the first actuating blade (4) comprises a first connecting bar (9) and/or a second connecting bar (10), wherein the second actuating blade (5) comprising the first connecting bar (9) and/or the second connecting bar (10).

5. Device according to claim 4, **characterized in that** the first connecting bar (9) has a guide pin (11) and the second connecting bar (10) has an elongated hole (12).

6. Device according to one of the previous claims 1 to 5, **characterized by** a protective cap (17) with pockets (18).

7. Device according to one of the previous claims 1 to 6, if dependent from claim 2, **characterized in that** the actuating blades (4, 5) are encasing the liquid intake (2) by a recess (20) in a contour pre-stressed manner.

8. Device according to claims 2 ,3 and 5, **characterized in that** the actuating blades (4, 5), which are movably connected via the film hinge (3), are fixed and movably held together via the connecting bars (9, 1 0) by the guide pin (11), which is arranged in the elongated hole (12) of the second connecting bar (10).

## Revendications

1. Dispositif de dosage du liquide provenant d'un récipient de liquide (2), dans lequel le récipient de liquide (2) comprend une vanne de sortie (1),
dans lequel
un système de dosage est fourni, dans lequel le système de dosage est constitué d'une première ailette d'actionnement (4) et d'une seconde ailette d'actionnement (5), lesquelles peuvent être disposées autour du récipient de liquide (2) et peuvent être déplacées l'une par rapport à l'autre de manière à comprimer le récipient de liquide (2), **caractérisé en ce que** la première ailette d'actionnement (4) comporte un premier piston de pression (28) et la seconde ailette d'actionnement (5) comporte un second piston de pression (32), dans lequel une chambre de distribution (27) est disposée de manière à pouvoir être repoussée par des pistons de pression (28, 32) montés mobiles l'un par rapport à l'autre, et les deux pistons de pression (28, 32) sont disposés de manière à pouvoir être remis à leur position initiale à l'aide d'un élément ressort (34) et/ou des ressorts partiels (39, 39.1).

2. Dispositif de dosage du liquide provenant d'un récipient de liquide (2), dans lequel le récipient de liquide (2) comprend une vanne de sortie (1),
dans lequel
un système de dosage est fourni, dans lequel le système de dosage est constitué d'une première ailette d'actionnement (4) et d'une seconde ailette d'actionnement (5), lesquelles peuvent être disposées autour du récipient de liquide (2) et
peuvent être déplacées l'une par rapport à l'autre de manière à comprimer le récipient de liquide (2), dans lequel une butée de dosage mécanique étant fournie, dans lequel le récipient de liquide (2) est enveloppé par un manchon (13) fendu du dispositif précontraint sur son contour, dans lequel
la première ailette d'actionnement (4) et la seconde ailette d'actionnement (5) comportent une paroi intérieure (6), dans lequel la première ailette d'actionnement (4) comporte un premier appui pour doigt (7) et la seconde ailette d'actionnement (5) comporte un second appui pour doigt (8), dans lequel deux brides d'actionnement pour doigt (14) sont disposées au niveau de l'extrémité supérieure du manchon (13) fendu, **caractérisé en ce qu'**une cheville (21) ronde conique est fournie, laquelle est située sur la paroi intérieure (6) des ailettes d'actionnement (4, 5), dans lequel lors de l'actionnement par l'intermédiaire des brides d'actionnement pour doigt (14) avec l'appui pour doigt (7, 8), un trou de ventilation (22) est fermé dans une paroi du récipient de liquide (2) et, dans la position de départ, le trou est à nouveau libéré pour la ventilation.

3. Dispositif selon la revendication 1 ou 2, **caractérisé en ce que** la première ailette d'actionnement (4) et la seconde ailette d'actionnement (5) sont reliées par l'intermédiaire d'une charnière pelliculaire (3) et peuvent pivoter l'une par rapport à l'autre.

4. Dispositif selon l'une quelconque des revendications précédentes 1 à 3, **caractérisé en ce que** la première ailette d'actionnement (4) comprend une première entretoise de liaison (9) et/ou une seconde entretoise de liaison (10), dans lequel la seconde aile d'actionnement (5) comprend la première entretoise de liaison (9) et/ou la seconde entretoise de liaison (10).

5. Dispositif selon la revendication 4 lorsqu'elle dépend de la revendication 2, **caractérisé en ce que** la première entretoise de liaison (9) comporte une goupille de guidage (11) et la seconde entretoise de liaison (10) comporte un trou oblong (12).

6. Dispositif selon l'une quelconque des revendications précédentes 1 à 5, **caractérisé par** un capuchon de protection (17) comportant des pochettes (18).

7. Dispositif selon l'une quelconque des revendications précédentes 1 à 6 lorsqu'elle dépend de la revendication 2, **caractérisé en ce que** les ailettes d'actionnement (4, 5) enveloppent le récipient de liquide (2) de manière précontrainte sur son contour par l'intermédiaire d'un évidement (20).

8. Dispositif selon les revendications 2, 3 et 5, **caractérisé en ce que** les étriers d'actionnement (4, 5), lesquels sont reliés de manière mobile par l'intermédiaire de la charnière pelliculaire (3), sont fixés l'un à l'autre et maintenus de manière mobile par l'intermédiaire des entretoises de liaison (9, 10) à l'aide de la goupille de guidage (11), laquelle est disposée dans le trou oblong (12) de la seconde entretoise de liaison (10).
